# FASCICULE DE BREVET EUROPEEN

(11) **EP 3 454 953 B1**
(45) Date de publication et mention de la délivrance du brevet: **08.07.2020**
(21) Numéro de dépôt: 17727294.5
(22) Date de dépôt: 11.05.2017
(51) Int. Cl.: A63B 33/00, A61F 9/02, G02C 5/04, G02C 5/12

(54) **LUNETTES**
BRILLE
EYEGLASSES

(30) Priorité: 12.05.2016 FR 1654250
(43) Date de publication de la demande: 20.03.2019
(73) Titulaire: Decathlon, 59650 Villeneuve d'Ascq (FR)
(72) Inventeur: CONAN, Mathieu, 59800 Lille (FR); HARPAGES, Antoine, 64210 Guethary (FR); WEINERT, André, 72655 Altdorf (DE); SAUMUREAU, Damien, 64310 Saint Pée sur Nivelle (FR)
(74) Mandataire: Sayettat, Julien Christian
(86) Numéro de dépôt international: PCT/FR2017/051126
(87) Numéro de publication internationale: WO 2017/194884

(56) Documents cités:
- WO-A2-2011/002194
- CN-Y- 2 401 911
- DE-A1- 3 934 163
- DE-U1- 9 005 624
- FR-A- 1 264 925
- GB-A- 2 326 078

## Description

L'invention concerne des lunettes comprenant deux unités optiques pourvues chacune d'une paroi transparente au travers de laquelle la vision s'effectue, lesdites unités optiques étant en outre équipées d'un dispositif de maintien desdites lunettes sur la tête d'un porteur.

Elle s'applique en particulier aux lunettes destinées à la pratique d'une activité aquatique telle que la natation. Dans ce cas, la périphérie arrière de chaque unité optique peut être équipée d'un joint destiné à épouser le visage du porteur, afin d'empêcher l'entrée d'eau entre ladite unité optique et l'œil dudit porteur.

Par ailleurs, le dispositif de maintien des lunettes sur la tête du porteur peut comprendre au moins une bande souple, notamment en matériau élastique, dont les extrémités sont attachées à un moyen, par exemple une fente ou une boucle de réglage, associé à un bord externe de respectivement une unité optique.

De façon conventionnelle, les unités optiques sont reliées entre elles par un pont de nez qui présente une partie centrale destinée à être disposée autour du nez du porteur, ainsi que deux parties latérales solidarisées chacune à un bord interne de respectivement une desdites unités.

Dans certaines versions, le positionnement des unités optiques par rapport au pont de nez est réglable, afin de pouvoir adapter les lunettes à la morphologie du visage du porteur, et ainsi d'améliorer le confort de portage, ou encore, dans le cas de lunettes adaptées à la pratique d'une activité aquatique, de garantir une bonne étanchéité entre lesdites lunettes et ledit visage.

Pour ce faire, on connaît des lunettes dans lesquelles chacune des unités optiques présente un bord interne équipé d'une fente au travers de laquelle une partie latérale du pont de nez est montée, ladite partie latérale présentant des crans qui sont répartis sur une partie de sa longueur et qui sont destinés à être engagés sélectivement dans ladite fente, selon le positionnement souhaité pour chacune des unités optiques.

Toutefois, cette solution ne donne pas entière satisfaction, en ce qu'elle nécessite un passage en force des crans dans les fentes, ce qui peut à long terme endommager le pont de nez, et ainsi réduire la durée de vie des lunettes. En outre, pour limiter l'encombrement du pont de nez, les parties latérales présentent généralement trois crans au maximum, ce qui limite les possibilités de réglage.

Pour pallier ces inconvénients, on a proposé des solutions dans lesquelles au moins une des unités optiques présente un bord interne équipé d'un conduit dans lequel une partie latérale du pont de nez est monté coulissante par l'intermédiaire d'un dispositif de réglage du positionnement de ladite unité optique par rapport audit pont de nez.

Ainsi, on connaît du document CN-2 401 911 des lunettes dans lesquelles le pont de nez présente deux filetages formés sur chacune de ses parties latérales, chacune desdites parties latérales étant vissable dans un taraudage formé dans le conduit d'un bord interne pour permettre le réglage attendu.

Par ailleurs, on connaît du document EP-0 891 788 des lunettes dans lesquelles des inserts sont disposés dans chacune des parties latérales pour en augmenter localement le diamètre, afin qu'il soit supérieur au diamètre intérieur du conduit du bord interne, tout en permettant le coulissement de ladite partie latérale relativement audit insert par traction manuelle.

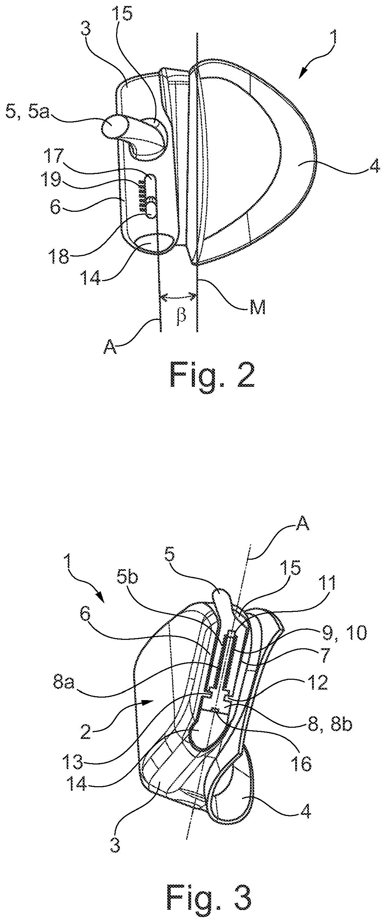

Le document FR-1 264 925 décrit des lunettes de vue comprenant deux unités optiques 17, 18 équipées d'une monture 1 de maintien sur la tête d'un porteur, ainsi qu'un pont de nez comprenant une partie centrale (formée par la branche de monture s'étendant horizontalement entre les unités 17, 18) et deux parties latérales 2, 2' destinées à être disposées de part et d'autre du nez dudit porteur, chaque partie latérale 2, 2' comprenant une plaquette 5 réglable en hauteur par rapport à ladite partie latérale par l'intermédiaire d'un dispositif comprenant :
- une armature 6 solidaire de la plaquette 5 qui, comme représenté sur la figure 3, forme un logement dans lequel une portion inférieure 3 de la partie latérale 2, 2' est mobile en translation verticale ;
- une vis filetée 9 fixée à l'armature 6 en s'étendant verticalement dans le logement, et montée coulissante dans un conduit taraudé 4 formé sur la portion inférieure 3 de la partie latérale 2, 2' pour permettre, par rotation de ladite vis dans ledit conduit, le réglage en hauteur de ladite plaquette par rapport à ladite partie latérale.

Le document WO 2011/002194 A2 décrit des lunettes de natation dont chaque unité optique 111 est pourvue d'un bord intérieur 141 avec un conduit 141 a dans lequel une partie latérale du pont de nez 130 est montée coulissante par l'intermédiaire d'un dispositif de réglage du positionnement de chacune desdites unités optiques par rapport audit pont de nez.

Toutefois, ces solutions ne donnent pas non plus entière satisfaction, en ce que le réglage qu'elles proposent reste relativement peu précis, en plus de manquer d'ergonomie.

L'invention vise à perfectionner l'art antérieur en proposant notamment des lunettes dans lesquelles le positionnement d'au moins une unité optique par rapport au pont de nez peut être réglé de manière très précise grâce à un dispositif simple, durable et ergonomique dans son utilisation.

A cet effet, l'invention propose des lunettes comprenant deux unités optiques équipées d'un dispositif de maintien desdites lunettes sur la tête d'un porteur, ainsi qu'un pont de nez qui relie lesdites unités en présentant une partie centrale destinée à être disposée autour du nez du porteur, au moins une desdites unités étant équipée d'un conduit dans lequel une partie latérale du pont de nez est montée coulissante par l'intermédiaire d'un dispositif de réglage du positionnement de ladite unité optique par rapport audit pont de nez, le dispositif de réglage comprenant un organe monté en rotation dans le conduit, ledit organe et la partie latérale du pont de nez comprenant des moyens d'accouplement qui sont agencés pour transformer la rotation dudit organe en translation relative entre ladite partie latérale et ledit conduit.

D'autres particularités et avantages de l'invention apparaîtront dans la description qui suit, faite en référence aux figures annexées, dans lesquelles :
- les figures 1a, 1b, 1c et 1d sont des représentations partielles de lunettes selon un mode de réalisation de l'invention, respectivement en perspective vue de dessus (figure 1a), en perspective vue de dessous (figure 1b), vue de face (figure 1c) et vue de dessous (figure 1d) ;
- la figure 2 est une représentation partielle en vue de côté interne d'une unité optique des lunettes selon les figures 1, une coupe longitudinale étant réalisée au niveau du pont de nez ;
- la figure 3 est une représentation partielle en perspective d'une unité optique des lunettes selon les figures 1, une coupe longitudinale étant réalisée au niveau du conduit pour rendre visibles l'accouplement de l'organe et de la partie latérale du pont de nez ;
- les figures 4a, 4b et 4c sont des représentations partielles en perspective des attaches destinées à solidariser entre elles les extrémités libres de deux bandes souples pour former un dispositif de maintien sur la tête du porteur pour les lunettes selon les figures 1, respectivement avec l'organe d'actionnement en position rangée (figure 4a), durant le déplacement dudit organe vers sa position d'utilisation (figure 4b) et avec ledit organe en position d'utilisation (figure 4c) ;
- la figure 5 est une représentation en perspective d'un moyen pour assurer le verrouillage réversible des attaches des figures 4 en position rangée de l'outil.

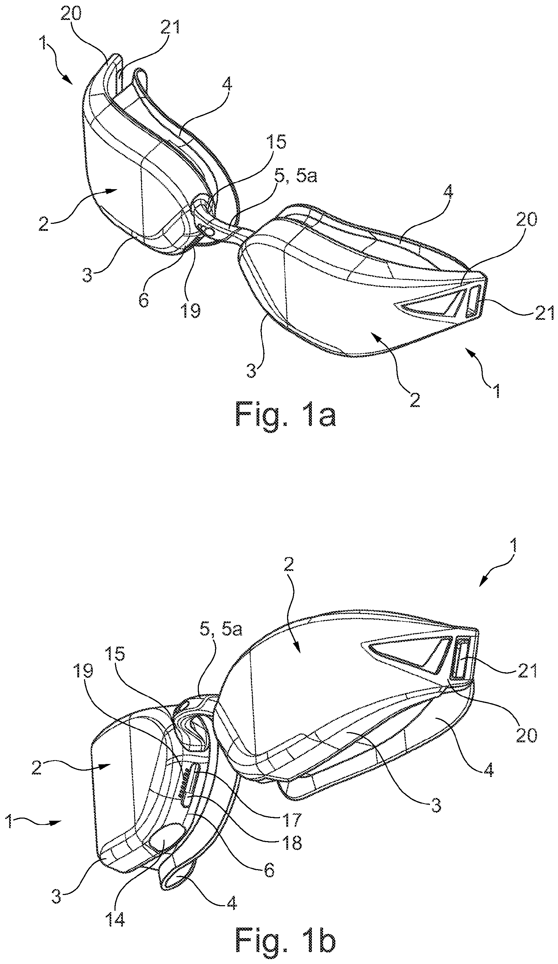

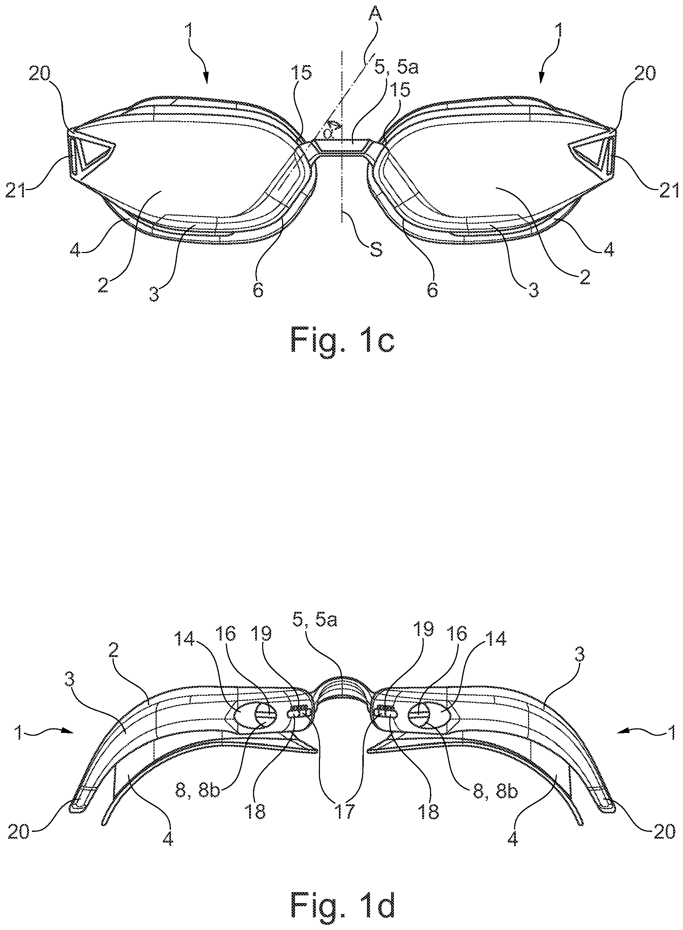

Dans cette description, les termes de positionnement dans l'espace sont pris en référence à la position des lunettes par rapport au visage d'un porteur ayant revêtu lesdites lunettes. En particulier, les termes « arrière » et « avant » sont relatifs à une disposition respectivement en regard du visage et opposée au visage, et les termes « interne » et « externe » sont relatifs à des dispositions respectivement proche et éloignée du nez du porteur. Par ailleurs, les termes « axial » et « radial » sont pris en référence à l'axe longitudinal A d'un conduit tel que représenté sur les figures 1c, 2 et 3, et correspondent à une direction respectivement parallèle et perpendiculaire à cet axe A.

En relation avec ces figures, on décrit ci-dessous des lunettes, notamment destinées à la pratique d'une activité aquatique telle que la natation.

Les lunettes comprennent deux unités optiques 1, qui présentent chacune une paroi transparente 2 agencée pour que la vision s'effectue au travers d'elle lorsque les lunettes sont revêtues par le porteur, par exemple en étant à base de verre ou d'un matériau polymère thermoplastique transparent. En particulier, les propriétés optiques de la paroi transparente 2 peuvent être agencées pour s'adapter à la vue du porteur et/ou à la luminosité ambiante.

Dans le mode de réalisation représenté, chaque unité optique 1 présente un cerclage 3 qui entoure au moins partiellement la paroi transparente 2, notamment afin de fournir une protection périphérique à ladite paroi transparente et/ou d'améliorer l'aspect esthétique des lunettes. Les cerclages 3 peuvent être réalisés en matériau rigide, par exemple à base d'un polymère thermoplastique, notamment d'une seule pièce avec la paroi transparente 2.

Par ailleurs, la paroi transparente 2 de chaque unité optique 1 présente une périphérie arrière qui est équipée d'un joint 4 destiné à épouser le visage du porteur, notamment afin d'empêcher la pénétration d'eau entre son œil et ladite paroi transparente. En particulier, le joint 4 peut être réalisé en matériau élastomère souple et être agencé pour venir en appui périphérique étanche autour de l'œil du porteur.

Les lunettes comprennent en outre un pont de nez 5 qui relie les unités optiques, ledit pont de nez présentant une partie centrale 5a destinée à être disposée autour du nez d'un porteur lorsqu'il revêt lesdites lunettes, ainsi que deux parties latérales 5b qui s'étendent de part et d'autre de ladite partie centrale et qui sont chacune associées à un bord interne 6 de respectivement une unité optique 1.

Selon un mode de réalisation, le pont de nez 5 présente une forme en U, la partie centrale 5a et les parties latérales 5b dudit pont de nez étant formées respectivement par le coude et par chacune des branches dudit U.

En particulier, le pont de nez 5 est agencé pour bien pouvoir positionner les unités optiques 1 sur les yeux du porteur, soit en présentant au moins une partie centrale 5a réalisée en matériau souple, par exemple à base de polymère élastomérique, afin de permettre, par sa déformabilité, une bonne adaptation à la morphologie du visage dudit porteur, soit en étant réalisé en matériau rigide avec une possibilité de réglage du positionnement d'au moins une unité 1 par rapport au pont de nez 5.

Pour ce faire, au moins une des unités optiques 1 est équipée d'un conduit 7 dans lequel une partie latérale 5b du pont de nez 5 est montée coulissante, notamment suivant l'axe longitudinal A dudit conduit, et ce par l'intermédiaire d'un dispositif de réglage du positionnement de ladite unité optique par rapport audit pont de nez.

Dans le mode de réalisation représenté, un tel conduit 7 est formé sur le bord interne 6 du cerclage 3 de chacune des unités optiques. En variante, le conduit 7 peut être formé sur une autre partie du cerclage 3, par exemple sur son bord supérieur ou inférieur.

Le pont de nez 5 présente deux parties latérales 5b qui sont montées coulissantes dans respectivement un des conduits 7 par l'intermédiaire d'un dispositif de réglage. Ainsi, il est possible de régler le positionnement relatif des unités optiques 1 sur le pont de nez 5, à savoir leur écartement notamment dans le cas d'un pont de nez 5 rigide, mais aussi leur positionnement l'une par rapport à l'autre. En variante, seules les parties latérales 5b du pont de nez 5 sont rigides, la partie centrale 5a étant souple pour que le réglage puisse contraindre la distance de déformation entre les unités optiques 1.

En particulier, en relation avec la figure 1c, l'axe longitudinal A de chaque conduit 7 peut former un angle α compris entre 30° et 60°, notamment inférieur à 45 ° et par exemple de l'ordre de 34°, avec un plan S de symétrie vertical des lunettes. Ainsi, le positionnement des unités optiques 1 entre elles et par rapport au visage est sensiblement conservé lors du réglage, tout en augmentant ou diminuant leur écartement.

En outre, l'axe longitudinal A de chaque conduit 7 peut former un angle β compris entre 1° et 10°, notamment de l'ordre de 5°, avec un plan médian M de l'unité optique 1 qui est sensiblement parallèle au visage du porteur. Ainsi, lors de leur écartement, les unités optiques 1 avancent et reculent légèrement en fonction du sens de réglage pour améliorer l'adaptation des lunettes à la morphologie du visage du porteur.

Le dispositif de réglage comprend un organe 8 monté en rotation dans le conduit 7, notamment suivant l'axe A, ledit organe et la partie latérale 5b du pont de nez 5 comprenant des moyens d'accouplement qui sont agencés pour transformer la rotation dudit organe en translation relative entre ladite partie latérale et ledit conduit.

En particulier, les moyens d'accouplement peuvent comprendre un filetage 9 et un taraudage 10 formés sur respectivement l'un parmi l'organe 8 et la partie latérale 5b du pont de nez 5. En relation avec la figure 3, l'organe 8 comprend une tige 8a dont la périphérie est pourvue d'un filetage 9, la partie latérale 5b comprenant un alésage 11 dans lequel ladite tige est disposée et qui est pourvu d'un taraudage 10 complémentaire dudit filetage.

L'organe 8 comprend en outre une tête 8b d'actionnement de sa rotation dans le conduit 7, ladite tête étant formée sur l'extrémité proximale de la tige 8a. Par ailleurs, le conduit 7 présente un rebord interne 12 au travers duquel l'organe 8 est engagé, ledit organe présentant une gorge 13 formée entre la tête d'actionnement 8b et l'extrémité proximale de la tige 8a, et dans laquelle ledit rebord est disposé en interférence axiale.

Ainsi, l'organe 8 est bloqué en translation dans le conduit 7 et, la partie latérale 5b étant bloquée en rotation dans ledit conduit, la rotation de l'organe 8 induit une translation relative entre ladite partie latérale et ledit conduit pour obtenir le réglage attendu.

Dans le mode de réalisation représenté, le conduit 7 présente une ouverture inférieure 14 par laquelle la tête d'actionnement 8b est accessible, ainsi qu'une ouverture supérieure 15 depuis laquelle s'étend la partie centrale 5a du pont de nez 5.

En outre, la tête d'actionnement 8b comprend une empreinte 16 agencée pour permettre l'actionnement en rotation de l'organe 8 au moyen d'un outil, par exemple sous la forme d'un tournevis.

Ainsi, grâce à ce dispositif, le porteur peut effectuer un réglage très précis du positionnement des unités optiques 1, ce qui lui permet d'adapter au mieux les lunettes à sa morphologie. Par ailleurs, le réglage peut être effectué de façon particulièrement simple et intuitive, en ce que l'actionnement de l'organe 8 permet directement le réglage, sans nécessiter de manipulation supplémentaire, notamment du pont de nez 5, ni induire de rotation dudit pont de nez.

Le conduit 7 présente une fente 17 qui débouche latéralement, la partie latérale 5b présentant un curseur 18 qui est disposé dans ladite fente. En particulier, le curseur 18 sert de butée de fin de course du déplacement de la partie latérale 5b dans le conduit 7, afin que ladite partie ne puisse pas sortir dudit conduit et ainsi reste accouplée à l'organe 8.

Pour améliorer la précision du réglage, le dispositif peut comprendre un moyen de visualisation de la position de la partie latérale 5b dans le conduit 7. Dans le mode de réalisation représenté, les lunettes comprennent des graduations 19 qui sont disposées le long de la fente 17 pour pouvoir quantifier la position de la partie latérale 5b dans le conduit 7, selon la position relative du curseur 18 par rapport auxdites graduations, ce qui permet au porteur d'effectuer plus facilement et plus rapidement un réglage adapté à sa morphologie, notamment entre deux utilisations des lunettes.

Pour assurer le maintien des lunettes sur la tête d'un porteur, les unités optiques 1 sont équipées d'un dispositif prévu à cet effet. Pour ce faire, en relation avec les figures, chaque unité optique 1 comprend un bord externe 20 équipé d'un moyen, notamment une cavité 21, pour permettre l'attache d'un tel dispositif.

Dans le mode de réalisation représenté, la cavité 21 est formée sur le bord externe 20 du cerclage 3 de chaque unité optique 1. En variante, notamment pour des unités optiques 1 dépourvues de cerclage, la cavité 21 peut être formée directement sur le bord externe de la paroi transparente 2.

En particulier, notamment pour des lunettes destinées à la pratique d'une activité aquatique, le dispositif de maintien des lunettes sur la tête du porteur peut comprendre au moins une bande souple, par exemple en matériau élastique, dont la longueur peut être réglable pour l'adapter à la taille de la tête du porteur.

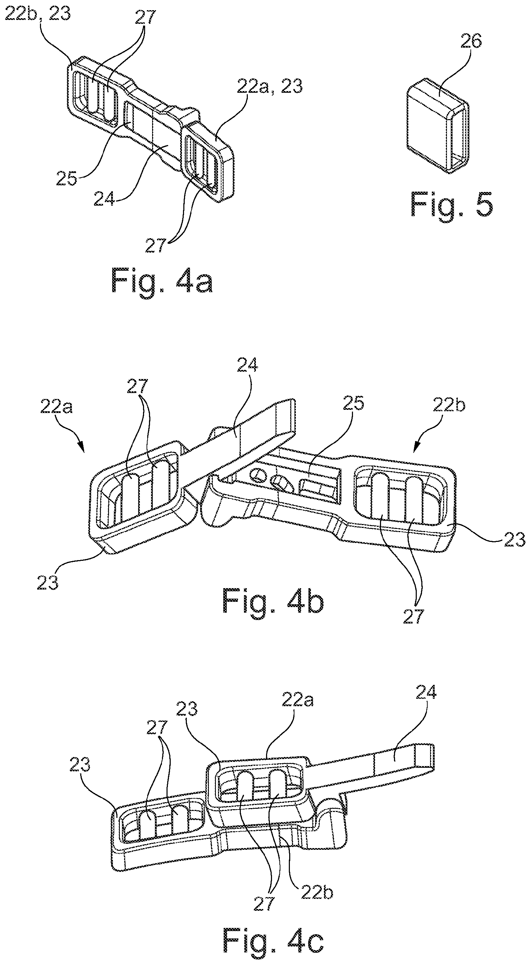

En relation avec les figures 4a-4c et 5, le dispositif de maintien comprend deux bandes (non représentées) qui présentent chacune une extrémité libre qui est équipée d'une attache 22a, 22b à l'autre bande, lesdites attaches comprenant chacune une boucle rigide 23 dans laquelle coulisse une desdites extrémités libres pour permettre l'ajustement de la longueur de la bande correspondante.

Les boucles 23 présentent chacune deux supports 27 autour desquelles l'extrémité libre d'une bande passe sélectivement, afin de permettre d'une part le réglage en longueur de ladite bande par coulissement de ladite extrémité dans un sens, et d'autre part d'empêcher le déréglage de ladite longueur en empêchant le coulissement de ladite extrémité dans le sens opposé.

De façon avantageuse, le dispositif de maintien intègre un outil 24 d'actionnement en rotation de l'organe 8 dans le conduit 7. Ainsi, le porteur dispose en permanence d'un tel outil 24 durant l'utilisation des lunettes, de sorte qu'il peut à tout moment ajuster le réglage de la longueur de la partie centrale 5a, et ce sans risque de perdre l'outil 24 entre deux utilisations.

Pour ce faire, le dispositif peut comprendre au moins une bande équipée d'une attache 22a qui porte un tel outil 24, ledit outil étant déployable, notamment depuis ladite attache, pour permettre son utilisation.

En relation avec les figures 4a-4c et 5, les attaches 22a, 22b de chacune des bandes de maintien portent respectivement l'outil 24 et un logement 25, notamment de forme complémentaire à celle dudit outil, lesdites attaches étant solidarisées entre elles de façon à permettre le déplacement dudit outil entre une position déployée d'utilisation (figure 4c) et une position rangée dans le logement 25 (figure 4a).

Dans le mode de réalisation représenté, l'outil 24 est monté en rotation sur l'autre attache 22b entre ses positions déployée et rangée, ce qui permet de passer d'une position à l'autre de manière simple et rapide.

En particulier, les attaches 22a, 22b sont solidarisées entre elles en rotation au niveau de l'axe de rotation de l'outil 24 entre ses positions déployée et rangée, ce qui permet de conserver le réglage en longueur des bandes quelle que soit la position dudit outil.

Par ailleurs, les attaches 22a, 22b comprennent un moyen de verrouillage desdites attaches dans la position rangée de l'outil 24, par exemple l'outil 24 peut être clipsé dans le logement 25 en position rangée. En outre, un passant 26 est monté coulissant entre les deux boucles 23 pour permettre, suivant sa position par rapport auxdites boucles, de garantir le verrouillage ou de permettre la libération du déplacement dudit outil entre ses deux positions (figure 5).

## Revendications

1. Lunettes comprenant deux unités optiques (1) équipées d'un dispositif de maintien desdites lunettes sur la tête d'un porteur, ainsi qu'un pont de nez (5) qui relie lesdites unités en présentant une partie centrale (5a) destinée à être disposée autour du nez du porteur, au moins une desdites unités étant équipée d'un conduit (7) dans lequel une partie latérale (5b) du pont de nez (5) est montée coulissante par l'intermédiaire d'un dispositif de réglage du positionnement de ladite unité optique par rapport audit pont de nez, le dispositif de réglage comprenant un organe (8) monté en rotation dans le conduit (7),
lesdites lunettes étant **caractérisées en ce que** ledit organe et la partie latérale (5b) du pont de nez (5) comprennent des moyens d'accouplement (9, 10) qui sont agencés pour transformer la rotation dudit organe en translation relative entre ladite partie latérale et ledit conduit.

2. Lunettes selon la revendication 1, **caractérisées en ce que** les moyens d'accouplement comprennent un filetage (9) et un taraudage (10) formés sur respectivement l'un parmi l'organe (8) et la partie latérale (5b) du pont de nez (5).

3. Lunettes selon l'une des revendications 1 ou 2, **caractérisées en ce que** l'organe (8) comprend une tête (8b) d'actionnement de sa rotation dans le conduit (7).

4. Lunettes selon la revendication 3, **caractérisées en ce que** le conduit (7) présente une ouverture inférieure (14) par laquelle la tête d'actionnement (8b) est accessible, ainsi qu'une ouverture supérieure (15) depuis laquelle s'étend la partie centrale (5a) du pont de nez (5).

5. Lunettes selon l'une des revendications 3 ou 4, **caractérisées en ce que** la tête d'actionnement (8b) comprend une empreinte (16) agencée pour permettre l'actionnement en rotation de l'organe (8) au moyen d'un outil (24).

6. Lunettes selon l'une quelconque des revendications 1 à 5, **caractérisées en ce que** le dispositif de réglage comprend un moyen de visualisation de la position de la partie latérale (5b) dans le conduit (7).

7. Lunettes selon l'une quelconque des revendications 1 à 6, **caractérisées en ce que** le conduit (7) présente une fente (17) qui débouche latéralement, la partie latérale (5b) présentant un curseur (18) qui est disposé dans ladite fente.

8. Lunettes selon l'une quelconque des revendications 1 à 7, **caractérisées en ce que** l'organe (8) est bloqué en translation dans le conduit (7), la partie latérale (5b) étant bloquée en rotation dans ledit conduit.

9. Lunettes selon l'une quelconque des revendications 1 à 8, **caractérisées en ce que** le dispositif de maintien intègre un outil (24) d'actionnement en rotation de l'organe (8) dans le conduit (7).

10. Lunettes selon la revendication 9, **caractérisées en ce que** le dispositif de maintien comprend au moins une bande équipée d'une attache (22a, 22b) portant un outil (24) qui est déployable pour permettre son utilisation.

11. Lunettes selon la revendication 10, **caractérisées en ce que** le dispositif de maintien comprend deux bandes équipées chacune d'une attache (22a, 22b) portant respectivement l'outil (24) et un logement (25), lesdites attaches étant solidarisées entre elles de façon à permettre le déplacement dudit outil entre une position déployée d'utilisation et une position rangée dans le logement (25).

12. Lunettes selon la revendication 11, **caractérisées en ce que** les attaches (22a, 22b) sont solidarisées entre elles en rotation au niveau d'un axe de rotation de l'outil (24) entre ses positions déployée et rangée.

13. Lunettes selon l'une des revendications 11 ou 12, **caractérisées en ce que** les attaches (22a, 22b) comprennent chacune une boucle rigide (23) dans laquelle coulisse une extrémité libre d'une bande pour permettre l'ajustement de la longueur de ladite bande.

14. Lunettes selon l'une quelconque des revendications 1 à 13, **caractérisées en ce que** chaque unité optique (1) comprend une paroi transparente (2) dont la périphérie arrière est équipée d'un joint (4) destiné à épouser le visage du porteur.

15. Lunettes selon l'une quelconque des revendications 1 à 14, **caractérisées en ce que** chaque unité optique (1) présente un bord interne (6) équipé d'un conduit (7) dans lequel une partie latérale (5b) du pont de nez (5) est montée coulissante par l'intermédiaire d'un dispositif de réglage pour pouvoir régler le positionnement relatif desdites unités sur ledit pont de nez.

16. Lunettes selon l'une quelconque des revendications 1 à 15, **caractérisées en ce que** le conduit (7) est formé sur un bord interne (6) d'une unité optique (1), ledit conduit présentant un axe longitudinal (A) formant un angle α compris entre 30° et 60° avec un plan (S) de symétrie vertical desdites lunettes.

17. Lunettes selon la revendication 16, **caractérisées en ce que** l'axe longitudinal (A) du conduit (7) forme un angle β compris entre 1° et 10 ° avec un plan médian (M) de l'unité optique (1).

## Patentansprüche

1. Brille, umfassend zwei optische Einheiten (1), die mit einer Haltevorrichtung der Brille auf dem Kopf eines Trägers ausgestattet sind, sowie einem Nasensteg (5), der die Einheiten verbindet, indem er einen zentralen Abschnitt (5a) aufweist, der zur Anordnung um die Nase des Trägers bestimmt ist, wobei mindestens eine der Einheiten mit einer Führung (7) ausgestattet ist, in der ein seitlicher Abschnitt (5b) des Nasenstegs (5) mittels einer Reguliervorrichtung der Positionierung der optischen Einheit in Bezug auf den Nasensteg gleitend angebracht ist, wobei die Reguliervorrichtung ein Organ (8) umfasst, das in der Führung (7) rotatorisch angebracht ist, wobei die Brille **dadurch gekennzeichnet ist, dass** das Organ und der seitliche Abschnitt (5b) des Nasenstegs (5) Kopplungsmittel (9, 10) umfassen, die ausgebildet sind, um die Rotation des Organs in relative Translation zwischen dem seitlichen Abschnitt und der Führung umzuwandeln.

2. Brille nach Anspruch 1, **dadurch gekennzeichnet, dass** die Kopplungsmittel ein Außengewinde (9) und ein Innengewinde (10) umfassen, die jeweils auf einem von dem Organ (8) und dem seitlichen Abschnitt (5b) des Nasenstegs (5) gebildet sind.

3. Brille nach einem der Ansprüche 1 oder 2, **dadurch gekennzeichnet, dass** das Organ (8) einen Betätigungskopf (8b) seiner Rotation in der Führung (7) umfasst.

4. Brille nach Anspruch 3, **dadurch gekennzeichnet, dass** die Führung (7) eine untere Öffnung (14) aufweist, durch die der Betätigungskopf (8b) erreichbar ist, sowie eine obere Öffnung (15), ab der sich der zentrale Teil (5a) des Nasenstegs (5) erstreckt.

5. Brille nach einem der Ansprüche 3 oder 4, **dadurch gekennzeichnet, dass** der Betätigungskopf (8b) einen Abdruck (16) umfasst, der ausgebildet ist, um die rotatorische Betätigung des Organs (8) mit einem Werkzeug (24) zu gestatten.

6. Brille nach einem der Ansprüche 1 bis 5, **dadurch gekennzeichnet, dass** die Reguliervorrichtung ein Visualisierungsmittel der Position des seitlichen Abschnitts (5b) in der Führung (7) umfasst.

7. Brille nach einem der Ansprüche 1 bis 6, **dadurch gekennzeichnet, dass** die Führung (7) einen Schlitz (17) aufweist, der seitlich mündet, wobei der seitliche Abschnitt (5b) einen Schieber (18) aufweist, der in dem Schlitz angeordnet ist.

8. Brille nach einem der Ansprüche 1 bis 7, **dadurch gekennzeichnet, dass** das Organ (8) in der Führung (7) translatorisch blockiert ist, wobei der seitliche Abschnitt (5b) in der Führung rotatorisch blockiert ist.

9. Brille nach einem der Ansprüche 1 bis 8, **dadurch gekennzeichnet, dass** die Haltevorrichtung ein Werkzeug (24) zur rotatorischen Betätigung des Organs (8) in der Führung (7) integriert.

10. Brille nach Anspruch 9, **dadurch gekennzeichnet, dass** die Haltevorrichtung mindestens ein Band umfasst, das mit einer Halterung (22a, 22b) ausgestattet ist, die ein Werkzeug (24) trägt, das ausklappbar ist, um seine Verwendung zu gestatten.

11. Brille nach Anspruch 10, **dadurch gekennzeichnet, dass** die Haltevorrichtung zwei Bänder umfasst, die jeweils mit einer Halterung (22a, 22b) ausgestattet sind, die jeweils ein Werkzeug (24) und eine Aufnahme (25) tragen, wobei die Halterungen derart fest miteinander verbunden sind, dass die Verlagerung des Werkzeugs zwischen einer ausgeklappten Benutzungsposition und einer in der Aufnahme (25) verstauten Position gestattet ist.

12. Brille nach Anspruch 11, **dadurch gekennzeichnet, dass** die Halterungen (22a, 22b) im Bereich einer Rotationsachse des Werkzeugs (24) zwischen ihrer ausgefahrenen und verstauten Position rotatorisch fest miteinander verbunden sind.

13. Brille nach einem der Ansprüche 11 oder 12, **dadurch gekennzeichnet, dass** jede der Halterungen (22a, 22b) eine starre Schnalle (23) umfasst, in der ein freies Ende eines Bandes gleitet, um die Anpassung der Länge des Bandes zu gestatten.

14. Brille nach einem der Ansprüche 1 bis 13, **dadurch gekennzeichnet, dass** jede optische Einheit (1) eine transparente Wand (2) umfasst, deren hinterer Umfang mit einer Dichtung (4) ausgestattet ist, die bestimmt ist, sich an das Gesicht des Trägers anzuschmiegen.

15. Brille nach einem der Ansprüche 1 bis 14, **dadurch gekennzeichnet, dass** jede optische Einheit (1) einen inneren Rand (6) aufweist, der mit einer Führung (7) ausgestattet ist, in der ein seitlicher Abschnitt (5b) des Nasenstegs (5) mittels einer Reguliervorrichtung gleitend montiert ist, um die relative Positionierung der Einheiten auf dem Nasensteg regulieren zu können.

16. Brille nach einem der Ansprüche 1 bis 15, **dadurch gekennzeichnet, dass** die Führung (7) auf einem inneren Rand (6) einer optischen Einheit (1) gebildet ist, wobei die Führung eine Längsachse (A) aufweist, die einen Winkel α zwischen 30° und 60° mit einer vertikalen Symmetrieebene (S) der Brille bildet.

17. Brille nach Anspruch 16, **dadurch gekennzeichnet, dass** die Längsachse (A) der Führung (7) einen Winkel β zwischen 1° und 10° mit einer Mittelebene (M) der optischen Einheit (1) bildet.

## Claims

1. Eyeglasses comprising two lens units (1) that are equipped with a retaining device for keeping said eyeglasses on a wearer's head, as well as a nose bridge (5) that connects said units while having a central portion (5a) intended to be positioned about the wearer's nose, at least one of said units being equipped with a duct (7) inside which a lateral portion (5b) of the nose bridge (5) is mounted such that it slides by means of a device for adjusting the positioning of said lens unit relative to said nose bridge, the adjusting device comprising a member (8) that is rotatably mounted in the duct (7), said eyeglasses being **characterized in that** said member and the lateral portion (5b) of the nose bridge (5) comprise coupling means (9, 10) that are designed to convert the rotation of said member into a relative translational movement between said lateral portion and said duct.

2. Eyeglasses according to claim 1, **characterised in that** the coupling means comprise a thread (9) and a tapping (10) formed on respectively one of either the member (8) and the lateral portion (5b) of the nose bridge (5).

3. Eyeglasses according to either claim 1 or claim 2, **characterised in that** the member (8) comprises an actuating head (8b) for actuating the rotation thereof in the duct (7) .

4. Eyeglasses according to claim 3, **characterised in that** the duct (7) has a lower opening (14) through which the actuating head (8b) can be accessed, as well as an upper opening (15) from which the central portion (5a) of the nose bridge (5) extends.

5. Eyeglasses according to either claim 3 or claim 4, **characterised in that** the actuating head (8b) comprises a recess (16) designed to allow the member (8) to be rotatably actuated by means of a tool (24).

6. Eyeglasses according to any of claims 1 to 5, **characterised in that** the adjusting device comprises a means for viewing the position of the lateral portion (5b) in the duct (7).

7. Eyeglasses according to any of claims 1 to 6, **characterised in that** the duct (7) has a slot (17) that opens out laterally, the lateral portion (5b) having a slider (18) that is arranged inside said slot.

8. Eyeglasses according to any of claims 1 to 7, **characterised in that** the translational movement of the member (8) in the duct (7) is blocked, whereby the rotational movement of the lateral portion (5b) in said duct is blocked.

9. Eyeglasses according to any of claims 1 to 8, **characterised in that** the retaining device incorporates a tool (24) for actuating the rotational movement of the member (8) in the duct (7).

10. Eyeglasses according to claim 9, **characterised in that** the retaining device comprises at least one strap equipped with a fastener (22a, 22b) that carries a tool (24) that is capable of being deployed to allow for the use thereof.

11. Eyeglasses according to claim 10, **characterised in that** the retaining device comprises two straps, each of which is equipped with a fastener (22a, 22b) that carries the tool (24) and a housing (25) respectively, said fasteners being secured to one another so as to allow said tool to be displaced between a deployed position of use and a stowed position inside the housing (25).

12. Eyeglasses according to claim 11, **characterised in that** the fasteners (22a, 22b) are secured to one another in rotation, at the level of a rotational axis of the tool (24) between the deployed and stowed positions thereof.

13. Eyeglasses according to either claim 11 or claim 12, **characterised in that** each of the fasteners (22a, 22b) comprises a rigid buckle (23) in which a free end of a strap slides in order to allow the length of said strap to be adjusted.

14. Eyeglasses according to any of claims 1 to 13, **characterised in that** each lens unit (1) comprises a transparent wall (2), the rear periphery whereof is equipped with a seal (4) intended to mould to the wearer's face.

15. Eyeglasses according to any of claims 1 to 14, **characterised in that** each lens unit (1) has an inner edge (6) equipped with a duct (7) in which a lateral portion (5b) of the nose bridge (5) is mounted such that it slides via an adjusting device so as to be able to adjust the relative positioning of said units on said nose bridge.

16. Eyeglasses according to any of claims 1 to 15, **characterised in that** the duct (7) is formed on an inner edge (6) of a lens unit (1), said duct having a longitudinal axis (A) forming an angle α that lies in the range from 30° to 60° with a vertical plane (S) of symmetry of said eyeglasses.

17. Eyeglasses according to claim 16, **characterised in that** the longitudinal axis (A) of the duct (7) forms an angle β that lies in the range from 1° to 10° with a median plane (M) of the lens unit (1).
